# EUROPÄISCHE PATENTSCHRIFT

(11) **EP 1 761 499 B1**
(45) Veröffentlichungstag und Bekanntmachung des Hinweises auf die Patenterteilung: **24.02.2010**
(21) Anmeldenummer: 05759346.9
(22) Anmeldetag: 11.06.2005
(51) Int. Cl.: C07D 231/14, A01N 43/56

(54) **1-METHYL-3-TRIFLUORMETHYL-PYRAZOL-4-CARBONSÄURE-(ORTHO-PHENYL)-ANILIDE UND IHRE VERWENDUNG ALS FUNGIZID**
1-METHYL-3-TRIFLUOROMETHYL-PYRAZOLE-4-CARBOXYLIC ACID (ORTHO-PHENYL)-ANILIDES AND TO USE THEREOF AS FUNGICIDE
1-METHYL-3-TRIFLUOROMETHYL-PYRAZOL-4-ACIDE CARBOXYLIQUE-(ORTHO-PHENYL)-ANILIDES ET LEUR UTILISATION COMME FONGICIDES

(30) Priorität: 18.06.2004 DE 102004029468
(43) Veröffentlichungstag der Anmeldung: 14.03.2007
(73) Patentinhaber: BASF SE, 67056 Ludwigshafen (DE)
(72) Erfinder: GEWEHR, Markus, 56288 Kastellaun (DE); MÜLLER, Bernd, 67227 Frankenthal (DE); GROTE, Thomas, 67157 Wachenheim (DE); GRAMMENOS, Wassilios, 67071 Ludwigshafen (DE); SCHWÖGLER, Anja, 68165 Mannheim (DE); RHEINHEIMER, Joachim, 67063 Ludwigshafen (DE); BLETTNER, Carsten, 68165 Mannheim (DE); SCHÄFER, Peter, 67308 Ottersheim (DE); SCHIEWECK, Frank, 67258 Hessheim (DE); WAGNER, Oliver, 67433 Neustadt (DE); WERNER, Frank, 67434 Neustadt (DE); RETHER, Jan, 67655 Kaiserslautern (DE); STRATHMANN, Siegfried, 67117 Limburgerhof (DE); STIERL, Reinhard, 67251 Freinsheim (DE); SCHERER, Maria, 76829 Godramstein (DE)
(86) Internationale Anmeldenummer: PCT/EP2005/006278
(87) Internationale Veröffentlichungsnummer: WO 2005/123689

(56) Entgegenhaltungen:
- EP-A- 0 589 301
- WO-A-00/14071
- WO-A-01/42223
- WO-A-03/070705
- PATENT ABSTRACTS OF JAPAN Bd. 1997, Nr. 09, 30. September 1997 (1997-09-30) -& JP 09 132567 A (MITSUI TOATSU CHEM INC), 20. Mai 1997 (1997-05-20) in der Anmeldung erwähnt

## Beschreibung

Die vorliegende Erfindung betrifft 1-Methyl-3-trifluormethyl-pyrazol-4-carbonsäure-(ortho-phenyl)-anilide der
Formel I 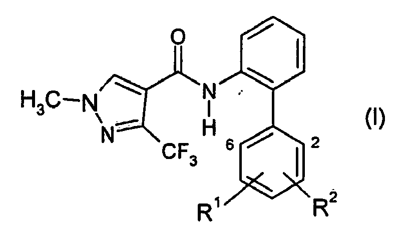 in der die Substituenten die folgende Bedeutung haben:
R¹ und R² unabhängig voneinander Halogen, C₁-C₆-Alkyl, Cyano, Nitro, Methoxy, Trifluormethoxy oder Difluormethoxy.

Außerdem betrifft die Erfindung ein Verfahren zur Bekämpfung von Schadpilzen mit den Verbindungen I und die Verwendung der Verbindungen I zur Herstellung fungizider Mittel.

1-Methyl-3-trifluormethyl-pyrazol-4-carbonsäure-(ortho-phenyl)-anilide sind aus der EP-A 0589301 bekannt, in der auch ein Verfahren zu deren Herstellung sowie eine Liste möglicher Mischungspartner aus der Reihe der Fungizide, Bakterizide, Akarizide, Nematizide oder Insektizide angegeben ist.

Aus der WO 01/42223 sind ebenfalls 1-Methyl-3-trifluormethyl-pyrazol-4-carbonsäure(ortho-phenyl)-anilide bekannt, die am Phenylring monosubstituiert sind.

Aus der JP 09 132567 sind 1-Methyl-3-trifluormethyl-pyrazol-4-carbonsäure(orthophenyl)-anilide bekannt, die am Phenylring durch Trifluormethyl substituiert sind.

Aus der WO03/070705 sind fungizide 1-Methyl-3-trifluoromethyl-pyrazol-4-carbonsäure(ortho-phenyl)-anilide bekannt, bei denen der Biphenylring im einen Ring einen Fluorsubstituenten und im anderen Phenylring zwei variable Substituenten trägt.

Aus der EP-A-589301 sind fungizide 1-Methyl-3-trifluoromethyl-pyrazol-4-carbonsäure(ortho-phenyl)-anilide bekannt, bei denen der Biphenylteil mehrere Substituenten tragen kann.

Die beschriebenen 1-Methyl-3-trifluormethyl-pyrazol-4-carbonsäure-anilide können jedoch, insbesondere bei niedrigen Aufwandmengen, nicht in vollem Umfang zufrieden stellen.

Aufgabe der vorliegenden Erfindungen war es, neue 1-Methyl-3-trifluormethyl-pyrazol-4-carbonsäure-anilide zur Verfügung zu stellen, die eine verbesserte fungizide Wirkung aufweisen, insbesondere auch bei niedrigen Aufwandmengen.

Demgemäss wurden die eingangs definierten Verbindungen der Formel I befunden.

Bevorzugt sind 1-Methyl-3-trifluormethyl-pyrazol-4-carbonsäure-(ortho-phenyl)-anilide der Formel I 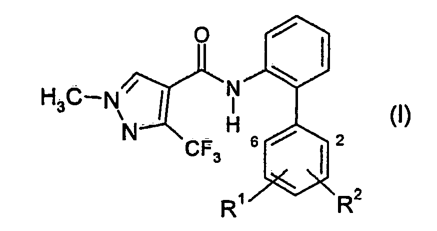 in der die Substituenten die folgende Bedeutung haben:
R¹ und R² unabhangig voneinander Fluor, Chlor, Cyano, Methyl, oder Methoxy.

Weiterhin sind Verbindungen der Formel I bevorzugt, bei denen R¹ und R² unabhängig voneinander Fluor, Chlor, Cyano oder Methoxy bedeuten.

Besonders bevorzugt sind Verbindungen der Formel I , bei denen R¹ und R² unabhängig voneinander Fluor oder Chlor bedeuten.
Ganz besonders bevorzugt sind Verbindungen der Formel I bei denen R¹ und R² in der 3- und 4-Position des Phenylrings stehen.

Von den erfindungsgemäßen Verbindungen I sind Verbindungen der Formeln Ia bis If bevorzugt, die in den folgenden Tabellen aufgeführt sind.

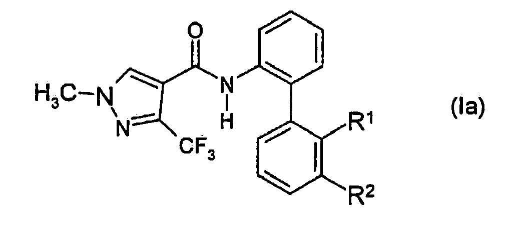

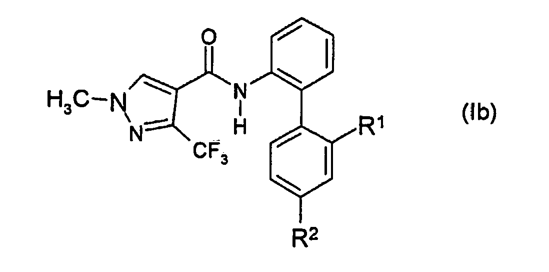

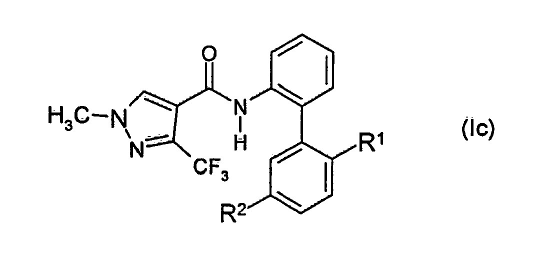

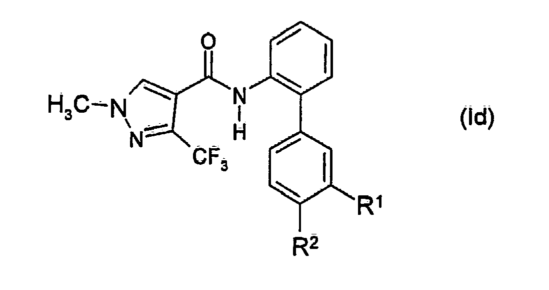

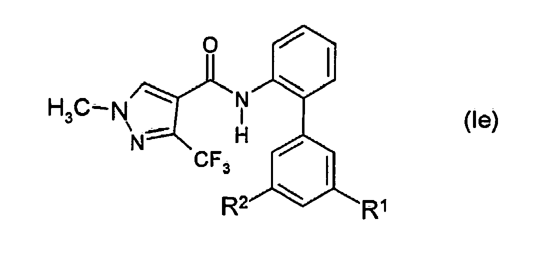

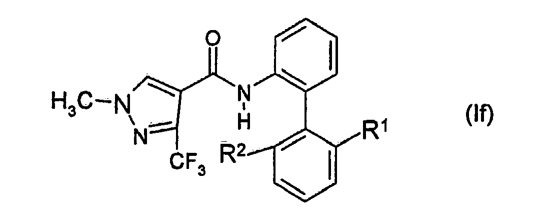

**Tabelle A**

| Nummer | R² |
|---|---|
| 1 | Fluor |
| 2 | Chlor |
| 3 | Brom |
| 4 | Iod |
| 5 | Methyl |
| 6 | Methoxy |
| 8 | Trifluormethoxy |
| 9 | Cyano |
| 10 | Nitro |
| 11 | Difluormethoxy |

### Tabelle 1:

### Verbindung 1.1-1.11

Verbindungen der Formel Ia, in denen R¹ Fluor bedeutet und R² für jeweils eine Bedeutung der Tabelle A steht.

### Tabelle 2:

### Verbindung 2.1-2.11

Verbindungen der Formel Ia, in denen R¹ Chlor bedeutet und R² für jeweils eine Bedeutung der Tabelle A steht.

### Tabelle 3:

### Verbindung 3.1-3.11

Verbindungen der Formel Ia, in denen R¹ Brom bedeutet und R² für jeweils eine Bedeutung der Tabelle A steht.

### Tabelle 4:

### Verbindung 4.1-4.11

Verbindungen der Formel Ia, in denen R¹ Jod bedeutet und R² für jeweils eine Bedeutung der Tabelle A steht.

### Tabelle 5:

### Verbindung 5.1-5.11

Verbindungen der Formel Ia, in denen R¹ Methyl bedeutet und R² für jeweils eine Bedeutung der Tabelle A steht.

### Tabelle 6:

### Verbindung 6.1-6.11

Verbindungen der Formel Ia, in denen R¹ Methoxy bedeutet und R² für jeweils eine Bedeutung der Tabelle A steht.

### Tabelle 8:

### Verbindung 8.1-8.11

Verbindungen der Formel Ia, in denen R¹ Trifluormethoxy bedeutet und R² für jeweils eine Bedeutung der Tabelle A steht.

### Tabelle 9:

### Verbindung 9.1-9.11

Verbindungen der Formel Ia, in denen R¹ Cyano bedeutet und R² für jeweils eine Bedeutung der Tabelle A steht.

### Tabelle 10:

### Verbindung 10.1-10.11

Verbindungen der Formel Ia, in denen R¹ Nitro bedeutet und R² für jeweils eine Bedeutung der Tabelle A steht.

### Tabelle 11:

### Verbindung 11.1-11.11

Verbindungen der Formel Ib, in denen R¹ Difluormethoxy bedeutet und R² für jeweils eine Bedeutung der Tabelle A steht.

### Tabelle 12:

### Verbindung 12.1-12.11

Verbindungen der Formel Ib, in denen R¹ Fluor bedeutet und R² für jeweils eine Bedeutung der Tabelle A steht.

### Tabelle 13:

### Verbindung 13.1-13.11

Verbindungen der Formel Ib, in denen R¹ Chlor bedeutet und R² für jeweils eine Bedeutung der Tabelle A steht.

### Tabelle 14:

### Verbindung 14.1-14.11

Verbindungen der Formel Ib, in denen R¹ Brom bedeutet und R² für jeweils eine Bedeutung der Tabelle A steht.

### Tabelle 15:

### Verbindung 15.1-15.11

Verbindungen der Formel Ib, in denen R¹ Jod bedeutet und R² für jeweils eine Bedeutung der Tabelle A steht.

### Tabelle 16:

### Verbindung 16.1-16.11

Verbindungen der Formel Ib, in denen R¹ Methyl bedeutet und R² für jeweils eine Bedeutung der Tabelle A steht.

### Tabelle 17:

### Verbindung 17.1-17.11

Verbindungen der Formel Ib, in denen R¹ Methoxy bedeutet und R² für jeweils eine Bedeutung der Tabelle A steht.

### Tabelle 19:

### Verbindung 19.1-19.11

Verbindungen der Formel Ib, in denen R¹ Trifluormethoxy bedeutet und R² für jeweils eine Bedeutung der Tabelle A steht.

### Tabelle 20:

### Verbindung 20.1 - 20.11

Verbindungen der Formel Ib, in denen R¹ Cyano bedeutet und R² für jeweils eine Bedeutung der Tabelle A steht.

### Tabelle 21:

### Verbindung 21.1 - 21.11

Verbindungen der Formel Ib, in denen R¹ Nitro bedeutet und R² für jeweils eine Bedeutung der Tabelle A steht.

### Tabelle 22:

### Verbindung 22.1 - 22.11

Verbindungen der Formel Ib, in denen R¹ Difluormethoxy bedeutet und R² für jeweils eine Bedeutung der Tabelle A steht.

### Tabelle 23:

### Verbindung 23.1 - 23.11

Verbindungen der Formel Ic, in denen R¹ Fluor bedeutet und R² für jeweils eine Bedeutung der Tabelle A steht.

### Tabelle 24:

### Verbindung 24.1-24.11

Verbindungen der Formel Ic, in denen R¹ Chluor bedeutet und R² für jeweils eine Bedeutung der Tabelle A steht.

### Tabelle 25:

### Verbindung 25.1-25.11

Verbindungen der Formel Ic, in denen R¹ Brom bedeutet und R² für jeweils eine Bedeutung der Tabelle A steht.

### Tabelle 26:

### Verbindung 26.1 - 26.11

Verbindungen der Formel Ic, in denen R¹ Jod bedeutet und R² für jeweils eine Bedeutung der Tabelle A steht.

### Tabelle 27:

### Verbindung 27.1 - 27.11

Verbindungen der Formel Ic, in denen R¹ Methyl bedeutet und R² für jeweils eine Bedeutung der Tabelle A steht.

### Tabelle 28:

### Verbindung 28.1 - 28.11

Verbindungen der Formel Ic, in denen R¹ Methoxy bedeutet und R² für jeweils eine Bedeutung der Tabelle A steht.

### Tabelle 30:

### Verbindung 30.1 - 30.11

Verbindungen der Formel Ic, in denen R¹ Trifluormethoxy bedeutet und R² für jeweils eine Bedeutung der Tabelle A steht.

### Tabelle 31:

### Verbindung 31.1-31.11

Verbindungen der Formel Ic, in denen R¹ Cyano bedeutet und R² für jeweils eine Bedeutung der Tabelle A steht.

### Tabelle 32:

### Verbindung 32.1 - 32.11

Verbindungen der Formel Ic, in denen R¹ Nitro bedeutet und R² für jeweils eine Bedeutung der Tabelle A steht.

### Tabelle 33:

### Verbindung 33.1-33.11

Verbindungen der Formel Ic, in denen R¹ Difluormethoxy bedeutet und R² für jeweils eine Bedeutung der Tabelle A steht.

### Tabelle 34:

### Verbindung 34.1 - 34.11

Verbindungen der Formel Id, in denen R¹ Fluor bedeutet und R² für jeweils eine Bedeutung der Tabelle A steht.

### Tabelle 35:

### Verbindung 35.1 -35.11

Verbindungen der Formel Id, in denen R¹ Chlor bedeutet und R² für jeweils eine Bedeutung der Tabelle A steht.

### Tabelle 36:

### Verbindung 36.1 - 36.11

Verbindungen der Formel Id, in denen R¹ Brom bedeutet und R² für jeweils eine Bedeutung der Tabelle A steht.

### Tabelle 37:

### Verbindung 37.1 - 37.11

Verbindungen der Formel Id, in denen R¹ Jod bedeutet und R² für jeweils eine Bedeutung der Tabelle A steht.

### Tabelle 38:

### Verbindung 38.1-38.11

Verbindungen der Formel Id, in denen R¹ Methyl bedeutet und R² für jeweils eine Bedeutung der Tabelle A steht.

### Tabelle 39:

### Verbindung 39.1 - 39.11

Verbindungen der Formel Id, in denen R¹ Methoxy bedeutet und R² für jeweils eine Bedeutung der Tabelle A steht.

### Tabelle 41:

### Verbindung 41.1 - 41.11

Verbindungen der Formel Id, in denen R¹ Trifluormethoxy bedeutet und R² für jeweils eine Bedeutung der Tabelle A steht.

### Tabelle 42

### Verbindung 42.1 - 42.11

Verbindungen der Formel Id, in denen R¹ Cyano bedeutet und R² für jeweils eine Bedeutung der Tabelle A steht.

### Tabelle 43:

### Verbindung 43.1 - 43.11

Verbindungen der Formel Id, in denen R¹ Nitro bedeutet und R² für jeweils eine Bedeutung der Tabelle A steht.

### Tabelle 44:

### Verbindung 44.1 - 44.11

Verbindungen der Formel Id, in denen R¹ Difluormethoxy bedeutet und R² für jeweils eine Bedeutung der Tabelle A steht.

### Tabelle 45:

### Verbindung 45.1-45.11

Verbindungen der Formel Ie, in denen R¹ Fluor bedeutet und R² für jeweils eine Bedeutung der Tabelle A steht.

### Tabelle 46:

### Verbindung 46.1 - 46.11

Verbindungen der Formel Ie, in denen R¹ Chlor bedeutet und R² für jeweils eine Bedeutung der Tabelle A steht.

### Tabelle 47:

### Verbindung 47.1-47.11

Verbindungen der Formel Ie, in denen R¹ Brom bedeutet und R² für jeweils eine Bedeutung der Tabelle A steht.

### Tabelle 48:

### Verbindung 48.1 - 48.11

Verbindungen der Formel Ie, in denen R¹ Jod bedeutet und R² für jeweils eine Bedeutung der Tabelle A steht.

### Tabelle 49:

### Verbindung 49.1 - 49.11

Verbindungen der Formel Ie, in denen R¹ Methyl bedeutet und R² für jeweils eine Bedeutung der Tabelle A steht.

### Tabelle 50:

### Verbindung 50.1 - 50.11

Verbindungen der Formel Ie, in denen R¹ Methoxy bedeutet und R² für jeweils eine Bedeutung der Tabelle A steht.

### Tabelle 52:

### Verbindung 52.1 - 52.11

Verbindungen der Formel Ie, in denen R¹ Trifluormethoxy bedeutet und R² für jeweils eine Bedeutung der Tabelle A steht.

### Tabelle 53:

### Verbindung 53.1 - 53.11

Verbindungen der Formel Ie, in denen R¹ Cyano bedeutet und R² für jeweils eine Bedeutung der Tabelle A steht.

### Tabelle 54:

### Verbindung 54.1 - 54.11

Verbindungen der Formel Ie, in denen R¹ Nitro bedeutet und R² für jeweils eine Bedeutung der Tabelle A steht.

### Tabelle 55:

### Verbindung 55.1-55.11

Verbindungen der Formel Ie, in denen R¹ Difluormethoxy bedeutet und R² für jeweils eine Bedeutung der Tabelle A steht.

### Tabelle 56:

### Verbindung 56.1-56.11

Verbindungen der Formel If, in denen R¹ Fluor bedeutet und R² für jeweils eine Bedeutung der Tabelle A steht.

### Tabelle 57:

### Verbindung 57.1 - 57.11

Verbindungen der Formel If, in denen R¹ Chlor bedeutet und R² für jeweils eine Bedeutung der Tabelle A steht.

### Tabelle 58:

### Verbindung 58.1 - 58.11

Verbindungen der Formel If, in denen R¹ Brom bedeutet und R² für jeweils eine Bedeutung der Tabelle A steht.

### Tabelle 59:

### Verbindung 59.1 - 59.11

Verbindungen der Formel If, in denen R¹ Jod bedeutet und R² für jeweils eine Bedeutung der Tabelle A steht.

### Tabelle 60:

### Verbindung 60.1-60.11

Verbindungen der Formel If, in denen R¹ Methyl bedeutet und R² für jeweils eine Bedeutung der Tabelle A steht.

### Tabelle 61:

### Verbindung 61.1-61.11

Verbindungen der Formel If, in denen R¹ Methoxy bedeutet und R² für jeweils eine Bedeutung der Tabelle A steht.

### Tabelle 63:

### Verbindung 63.1-63.11

Verbindungen der Formel If, in denen R¹ Trifluormethoxy bedeutet und R² für jeweils eine Bedeutung der Tabelle A steht.

### Tabelle 64:

### Verbindung 64.1 - 64.11

Verbindungen der Formel If, in denen R¹ Cyano bedeutet und R² für jeweils eine Bedeutung der Tabelle A steht.

### Tabelle 65:

### Verbindung 65.1-65.11

Verbindungen der Formel If, in denen R¹ Nitro bedeutet und R² für jeweils eine Bedeutung der Tabelle A steht.

### Tabelle 66:

### Verbindung 66.1-66.11

Verbindungen der Formel If, in denen R¹ Difluormethoxy bedeutet und R² für jeweils eine Bedeutung der Tabelle A steht.

Im Rahmen der vorliegenden Erfindung steht Halogen für Fluor, Chlor, Brom und Jod und insbesondere für Fluor und Chlor.

Der Ausdruck "Alkyl" umfasst geradkettige und verzweigte Alkylgruppen. Vorzugsweise handelt es sich dabei um geradkettige oder verzweigte C₁-C₆-Alkylgruppen. Beispiele für Alkylgruppen sind Alkyl wie insbesondere Methyl, Ethyl, Propyl, 1-Methylethyl, Butyl, 1-Methylpropyl, 2-Methylpropyl 1,1-Dimethylethyl.

Verfahren zur Herstellung der Verbindungen der allgemeinen Formel F sind aus der EP-A 0 589 301 bekannt.

Beispielsweise setzt man das 1-Methyl-3-trifluormethyl-pyrazolcarbonsäure-halogenide der Formel II mit einem Anilin der Formel III zu den Verbindungen der Formel I um:

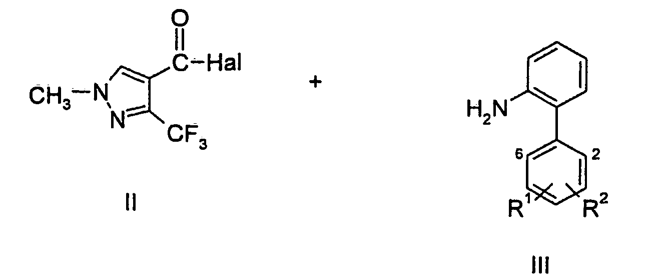

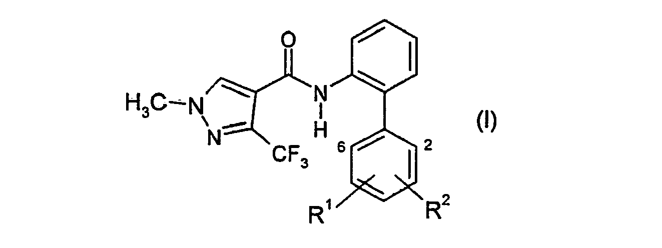

Der Rest Hal in der Formel II steht für ein Halogenatom wie Chlor, Brom und Jod, insbesondere Chlor oder Brom.
Diese Umsetzung erfolgt üblicherweise bei Temperaturen von -20 °C bis 100 °C, vorzugsweise 0 °C bis 50 °C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Triethylamin und Pyridin verwendet.

Die Basen werden im allgemeinen in äquimolarem Mengen bezogen auf die Verbindung II eingesetzt. Sie können aber auch in einem Überschuß von 5 mol-% bis 30 mol-%, vorzugsweise 5 mol-% bis 10 mol-%, oder- im Falle der Verwendung von tertiären Aminen - gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die erfindungsgemäßen Verbindungen I können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenylisopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthiotetrahydrophthalimid, N-Trichlormethylthio-phthalimid,
- N-Dichlorfluormethylthio-N',N'-dimethyl-N-phenyl-schwefelsäure- diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-lod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, a-(2-Chlorphenyl)-a-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[a-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[a-(2-phenoxyphenyl)]-acetamid, Methyl-E-methoxyimino-[a-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I zeichnen sich durch eine hervorragende Wirkung gegen ein breites Spektrum von pflanzenpathogenen Pilzen, insbesondere aus der Klasse der Ascomyceten, Basidiomyceten, Phycomyceten und Deuteromyceten aus. Sie sind z.T. systemisch wirksam und können daher auch als Blatt- und Bodenfungizide eingesetzt werden. Sie können auch zur Saatgutbehandlung verwendet werden.

Besondere Bedeutung haben sie für die Bekämpfung einer Vielzahl von Pilzen an verschiedenen Kulturpflanzen wie Baumwolle, Gemüsepflanzen (z.B. Gurken, Bohnen, Tomaten, Kartoffeln und Kürbisgewächse), Gerste, Gras, Hafer, Bananen, Kaffee, Mais, Obstpflanzen, Reis, Roggen, Soja, Wein, Weizen, Zierpflanzen, Zuckerrohr sowie an einer Vielzahl von Samen.

Insbesondere eignen sie sich zur Bekämpfung der folgenden pflanzenpathogenen Pilze: Blumeria graminis (echter Mehltau) an Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinera (Grauschimmel) an Erdbeeren, Gemüse, Zierpflanzen und Reben, Cercospora arachidicola an Erdnüssen, Pseudocercosporella herpotrichoides an Weizen und Gerste, Pyricularia oryzae an Reis, Phytophthora infestans an Kartoffeln und Tomaten, Plasmopara viticola an Reben, Pseudoperonospora-Arten in Hopfen und Gurken, Alternaria-Arten an Gemüse und Obst, Mycosphaerella-Arten in Bananen sowie Fusarium- und Verticillium-Arten.

Die Aufwandmengen der erfindungsgemäßen Verbindungen der Formel I liegen, vor allem bei landwirtschaftlichen Kulturflächen, je nach Art des gewünschten Effekts bei 0,01 bis 8 kg/ha, vorzugsweise 0,1 bis 5 kg/ha, insbesondere 0,1 bis 3,0 kg/ha.

Insbesondere liegen die Aufwandmengen dabei für die Verbindung I bei 0,01 bis 1 kg/ha, vorzugsweise 0,05 bis 0,5 kg/ha, insbesondere 0,05 bis 0,3 kg/ha.

Bei der Saatgutbehandlung werden im allgemeinen Aufwandmengen an Mischung von 0,001 bis 250 g/kg Saatgut, vorzugsweise 0,01 bis 100 g/kg, insbesondere 0,01 bis 50 g/kg verwendet.

Sofern für Pflanzen pathogene Schadpilze zu bekämpfen sind, erfolgt die Applikation der Verbindung I durch Besprühen oder Bestäuben der Samen, der Pflanzen oder der Böden vor oder nach der Aussaat der Pflanzen oder vor oder nach dem Auflaufen der Pflanzen.

Die erfindungsgemäßen fungiziden Verbindungen I können beispielsweise in Form von direkt versprühbaren Lösungen, Pulver und Suspensionen oder in Form von hochprozentigen wäßrigen, öligen oder sonstigen Suspensionen, Dispersionen, Emulsionen, Öldispersionen, Pasten, Stäubemitteln, Streumitteln oder Granulaten aufbereitet und durch Versprühen, Vernebeln, Verstäuben, Verstreuen oder Gießen angewendet werden. Die Anwendungsform ist abhängig vom Verwendungszweck; sie soll in jedem Fall eine möglichst feine und gleichmäßige Verteilung der erfindungsgemäßen Mischung gewährleisten.

Die Formulierungen werden in an sich bekannter Weise hergestellt, z.B. durch Zugabe von Lösungsmitteln und/oder Trägerstoffen gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole oder Fettalkoholglycolethern, Kondensationsprodukte von sulfoniertem Naphthalin und seinen Derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- oder Tributylphenylpolyglycolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglycoletheracetat, Sorbitester, Lignin-Sulfitablaugen oder Methylcellulose in Betracht.

Pulver Streu- und Stäubemittel können durch Mischen oder gemeinsames Vermahlen der Verbindung I mit einem festen Trägerstoff hergestellt werden.

Granulate (z.B. Umhüllungs-, Imprägnierungs- oder Homogengranulate) werden üblicherweise durch Bindung des Wirkstoffs oder der Wirkstoffe an einen festen Trägerstoff hergestellt.

Als Füllstoffe bzw. feste Trägerstoffe dienen beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, sowie Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen 0,1 bis 95 Gew.-%, vorzugsweise 0,5 bis 90 Gew.-% der Verbindung I.
Die Wirkstoffe werden dabei in einer Reinheit von 90% bis 100%, vorzugsweise 95% bis 100% (nach NMR- oder HPLC-Spektrum) eingesetzt.

Beispiele für Formulierungen sind: 1. Produkte zur Verdünnung in Wasser
A) Wasserlösliche Konzentrate (SL)
   10 Gew.-Teile der Wirkstoffe werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
B) Dispergierbare Konzentrate (DC)
   20 Gew.-Teile der Wirkstoffe werden in Cyclohexanon unter Zusatz eines Dispergiermittels z.B. Polyvinylpyrrolidon gelöst. Bei Verdünnung in Wasser ergibt sich eine Dispersion.
C) Emulgierbare Konzentrate (EC)
   15 Gew.-Teile der Wirkstoffe werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
D) Emulsionen (EW, EO)
   40 Gew.-Teile der Wirkstoffe werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
E) Suspensionen (SC, OD)
   20 Gew.-Teile der Wirkstoffe werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
F) Wasserdispergierbare und wasserlösliche Granulate (WG, SG)
   50 Gew.-Teile der Wirkstoffe werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
G) Wasserdispergierbare und wasserlösliche Pulver (WP, SP)
   75 Gew.-Teile der Wirkstoffe werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

H) Stäube (DP)
   5 Gew.Teile der Wirkstoffe werden fein gemahlen und mit 95 % feinteiligem Kaolin innig vermischt. Man erhält dadurch ein Stäubmittel.
I) Granulate (GR, FG, GG, MG)
   0.5 Gew-Teile der Wirkstoffe werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
J) ULV- Lösungen (UL)
   10 Gew.-Teile der Wirkstoffe werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 1 zugemischt werden.

Die Anwendung der Verbindung I oder der entsprechenden Formulierungen erfolgt so, daß man die Schadpilze, deren Lebensraum oder die von ihnen freizuhaltenden Pflanzen, Samen, Böden, Flächen, Materialien oder Räume mit einer fungizid wirksamen Menge der Verbindung I behandelt.

Die Anwendung kann vor oder nach dem Befall durch die Schadpilze erfolgen.

### Herstellungsbeispiele

### Beispiel 1

### 1.) Synthese von ortho-(3,4-Dichlophenyl)-anilin

Zu einer Lösung von 20,47 g 2-Brom-anilin, 24,98 g 3,4-Dichlorphenylboronsäure und 25,23 g Natriumcarbonat in einer Mischung von 150 ml Wasser und 450 ml Ethylenglycoldimethylether wurden 0,14 g Tetrakistriphenylphosphin-Palladium(O) gegeben. Man rührte 48 unter Rückfluß. Der Ansatz wurde im Vakuum eingeengt. Der Rückstand wurde mit Methyl-tert-butylether aufgenommen, einmal mit Natriumhydrogencarbonatlösung und viermal mit Wasser gewaschen, über Natriumsulfat getrocknet und im Vakuum eingeengt. Nach chromatografischer Reinigung mit einer Mischung von Toluol und Cyclohexan (1:2) erhielt man 15,5 g des Produktes als hellgelbes Pulver.

### 2.) Synthese von 1-Methyl-3-trifluormethyl-N-(ortho-(3,4-dichlorphenyl)phenyl)-pyrazol-4-carbonsäureamid.

In 10 ml Tuluol wurden 0,32 g 1-Methyl-3-trifluormethyl-pyrazol-4-carbonsäurechorid, 0,36 g ortho-(3,4-Dichlorphenyl)anilin und 0,23 g Triethylamin gelöst. Nachdem 4 h bei Raumtemperatur gerührt wurde, wurden 20 ml Methyl-tert-butylether zugegeben, zweimal mit 5%-iger Salzsäure, zweimal mit 5%iger Natronlauge und einmal mit Natriumchloridlösung gewaschen. Die organische Phase wurde über Natriumsulfat getrocknet und im Vakuum eingeengt. Nach chromatografischer Reinigung mit einer Mischung von Toluol und Methyl-tert-butylether erhielt man 0,32 g des Produktes als farbloses Pulver. Fp = 131-133°C.

**Tabelle 67**

| | | | |
|---|---|---|---|
| 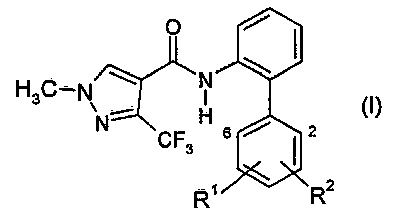 | | | |

| **Verb.** | **R²** | **R¹** | **Fp** |
|---|---|---|---|
| 67.1 | 3-Cl | 4-Cl | 131-133°C |
| 67.2 | 3-Cl | 4-F | 133-134°C |
| 67.3 | 2-Cl | 4-Cl | 136-140°C |
| 67.4 | 3-F | 4-F | 126-128°C |
| 67.5 | 2-Cl | 5-Cl | 101-105°C |
| 67.6 | 2-F | 5-F | 110-112°C |
| 67.7 | 2-F | 4-Cl | 136-138°C |
| 67.8 | 2-CH₃ | 4-Cl | 130-131°C |
| 67.9 | 3-CH₃ | 4-Cl | 109-111°C |
| 67.10 | 2-CH₃ | 4-F | 125-126°C |
| 67.11 | 3-CH₃ | 4-F | 126-127°C |
| 67.12 | 3-F | 4-Cl | 149-150°C |
| 67.13 | 2-F | 4-F | 100-102°C |
| 67.14 | 2-F | 4-OCH₃ | 102-104°C |
| 67.15 | 2-F | 6-F | 140-143°C |

| | | | **HPLC-Retentionszeit [min]** |
|---|---|---|---|
| 67.16 | 3-CH₃ | 5-CH₃ | 3,71 |
| 67.17 | 3-NO₂ | 4-Cl | 3,36 |
| 67.19 | 3-CH₃ | 4-CH₃ | 2,99 |
| 67.21 | 3-CH₃ | 4-OCH₃ | 3,55 |
| 67.22 | 3-OCH₃ | 4-OCH₃ | 3,03 |
| 67.23 | 3-F | 4-OCH₃ | 3,26 |
| 67.24 | 3-OCH₃ | 4-Cl | 3,46 |

### Anwendungsbeispiele

Die Wirkstoffe wurden als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Anwendungsbeispiel 1 - Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 2 - 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1.7 x 10⁶ Sporen/ml in einer 2 %igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

| **Verbindung** | **Struktur** | **Befall in % bei** **4ppm** **bei BOTRCI P1** |
|---|---|---|
| Verb. 37 der EP-A 05 89 301 | 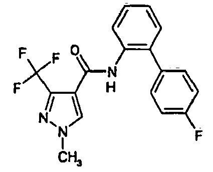 | 70 |
| Verb. 67.4 | 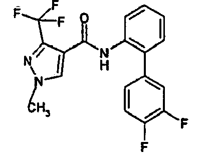 | 40 |
| Unbehandelt | | 90 |

### Anwendungsbeispiel 2 - Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropf-nässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

| **Verbindung** | **Struktur** | **Befall in % bei** **4ppm** **bei PUCCRT K1** |
|---|---|---|
| Verb. 37 der EP-A 05 89 301 | 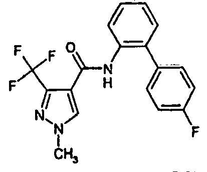 | 30 |
| Verb. 67.4 | 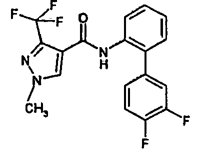 | 5 |
| Unbehandelt | | 90 |

### Anwendungsbeispiel 1 - Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropf-nässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

| **Verbindung** | **Struktur** | **Befall in % bei** **250ppm** **bei PUCCRT K1** |
|---|---|---|
| Verb. 38 der EP-A 05 89 301 | 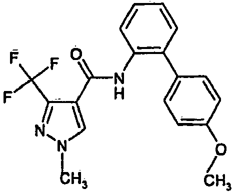 | 60 |
| Verb. 67.14 | 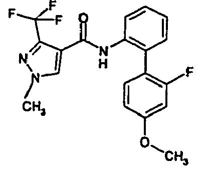 | 7 |
| Unbehandelt | | 90 |

### Anwendungsbeispiel 1 - Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 2 - 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1.7 x 10⁶ Sporen/ml in einer 2 %igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

| **Verbindung** | **Struktur** | **Befall in % bei** **250ppm** **bei BOTRCI P1** |
|---|---|---|
| Verb. 41 der EP-A 05 89 301 | 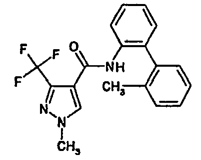 | 25 |
| Verb. 67.8 | 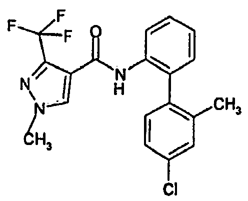 | 0 |
| Unbehandelt | | 90 |

### Anwendungsbeispiel 2 - Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropf-nässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

| **Verbindung** | **Struktur** | **Befall in % bei** **250ppm** **bei PUCCRT K1** |
|---|---|---|
| Verb. 41 der EP-A 05 89 301 | 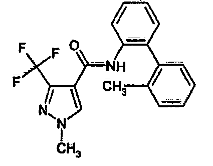 | 60 |
| Verb. 67.8 | 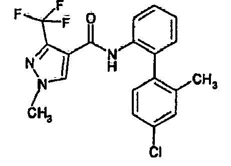 | 0 |
| Unbehandelt | | 90 |

## Patentansprüche

1. 1-Methyl-3-trifluormethyl-pyrazol-4-carbonsäure(ortho-phenyl)-anilide der Formel I 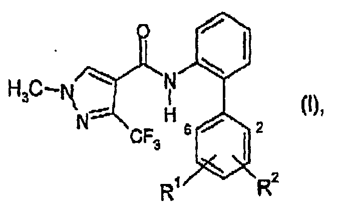 in der die Substituenten die folgende Bedeutung haben:
R¹ Cyano, Nitro, Halogen, C₁-C₆-Alkyl, Methoxy, Trifluormethoxy oder Difluormethoxy;
R² Cyano, Nitro, Halogen, C₁-C₆-Alkyl, Methoxy, Trifluormethoxy oder Difluormethoxy.

2. Anilide der Formel I nach Anspruch 1, wobei R¹ und R² unabhängig voneinander für Cyano, Fluor, Chlor, Methyl oder Methoxy stehen.

3. Anilide der Formel I nach Anspruch 1 oder 2, wobei R¹ und R² unabhängig voneinander für Cyano, Fluor, Chlor oder Methoxy stehen.

4. Anilide der Formel I nach Anspruch 1 oder 2, wobei R¹ und R² unabhängig voneinander für Fluor oder Chlor stehen.

5. Anilide der Formel I nach einem der Ansprüche 1 bis 5, wobei die Substituenten R¹ und R² in der 3- und 4-Position des Phenylringes stehen.

6. Verfahren zur Bekämpfung von Schadpilzen, **dadurch gekennzeichnet, dass** man die Schadpilze, deren Lebensraum oder die von ihnen freizuhaltenden Pflanzen, Samen, Böden, Flächen, Materialien oder Räume mit einem 1-Methyl-3-trifluormethyl-pyrazol-4-carbonsäure-(ortho-phenyl)-anilid der Formel I gemäß einem der Ansprüche 1 bis 5 behandelt.

7. Fungizide Mittel, enthaltend ein 1-Methyl-3-trifluormethyl-pyrazol-4-carbonsäure-(ortho-phenyl)-anilid der Formel I gemäß einem der Ansprüche 1 bis 5 sowie einen festen oder flüssige Träger.

8. Verwendung der Verbindungen I gemäß einem der Ansprüche 1 bis 5 zur Bekämpfung von pflanzenpathogenen Schadpilzen.

## Claims

1. An N-(ortho-phenyl)-1-methyl-3-trifluoromethylpyrazole-4-carboxanilide of the formula I 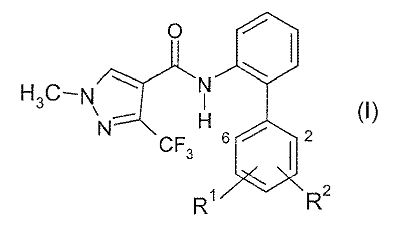 in which the substituents are as defined below:
R¹ is cyano, nitro, halogen, C₁-C₆-alkyl, methoxy, trifluoromethoxy or difluoromethoxy;
R² is cyano, nitro, halogen, C₁-C₆-alkyl, methoxy, trifluoromethoxy or difluoromethoxy.

2. The anilide of the formula I according to claim 1 in which R¹ and R² independently of one another are cyano, fluorine, chlorine, methyl or methoxy.

3. The anilide of the formula I according to claim 1 or 2 in which R¹ and R² independently of one another are cyano, fluorine, chlorine or methoxy.

4. The anilide of the formula I according to claim 1 or 2 in which R¹ and R² independently of one another are fluorine or chlorine.

5. The anilide of the formula I according to any of claims 1 to 5 in which the substituents R¹ and R² are located in the 3- and 4-positions of the phenyl ring.

6. A method for controlling harmful fungi wherein the harmful fungi, their habitat or the plants, seeds, soils, areas, materials or spaces to be kept free from them are treated with an N-(ortho-phenyl)-1-methyl-3₋trifluoromethylpyrazole-4-carboxanilide of the formula I according to any of claims 1 to 5.

7. A fungicidal composition comprising an N-(ortho-phenyl)-1-methyl-3-trifluoromethylpyrazole-4-carboxanilide of the formula I according to any of claims 1 to 5 and a solid or liquid carrier.

8. The use of the compounds I according to any of claims 1 to 5 for controlling phytopathogenic harmful fungi.

## Revendications

1. (Ortho-phényl)-anilides d'acide 1-méthyl-3-trifluorométhyl-pyrazole-4-carboxylique de formule I 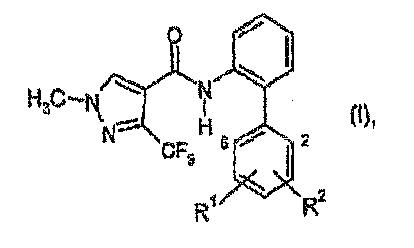 dans laquelle les substituants ont les significations suivantes :
R¹ représente un atome d'halogène ou un groupe cyano, nitro, alkyle en C₁-C₆, méthoxy, trifluorométhoxy ou difluorométhoxy ;
R² représente un atome d'halogène ou un groupe cyano, nitro, alkyle en C₁-C₆, méthoxy, trifluorométhoxy ou difluorométhoxy.

2. Anilides de formule I selon la revendication 1, dans lesquels R¹ et R² représentent, indépendamment l'un de l'autre, un atome de fluor ou de chlore ou un groupe cyano, méthyle ou méthoxy.

3. Anilides de formule I selon la revendication 1 ou 2, dans lesquels R¹ et R² représentent, indépendamment l'un de l'autre, un atome de fluor ou de chlore ou un groupe cyano ou méthoxy.

4. Anilides de formule I selon la revendication 1 ou 2, dans lesquels R¹ et R² représentent, indépendamment l'un de l'autre, un atome de fluor ou de chlore.

5. Anilides de formule I selon l'une quelconque des revendications 1 à 5, dans lesquels les substituants R¹ et R² se trouvent en la position 3 et la position 4 du noyau phényle.

6. Procédé pour la lutte contre des champignons nuisibles, **caractérisé en ce qu'**on traite par un (ortho-phényl)-anilide d'acide 1-méthyl-3-trifluorométhyl-pyrazole-4-carboxylique de formule I selon une ou plusieurs des revendications 1 à 5, les champignons, leur habitat ou les plantes, semences, sols surfaces, matériaux ou espaces à maintenir hors d'atteinte par ceux-ci.

7. Produit fongicide, contenant un (ortho-phényl)-anilide d'acide 1-méthyl-3-trifluorométhyl-pyrazole-4-carboxylique de formule I selon l'une quelconque des revendications 1 à 5, ainsi qu'une substance de support solide ou liquide.

8. Utilisation des composés I selon l'une quelconque des revendications 1 à 5, pour la lutte conte des champignons nuisibles phytopathogènes.
